(19) Europäisches Patentamt
European Patent Office
Office européen des brevets

(11) Veröffentlichungsnummer: **0 009 776**
**B1**

# (12) EUROPÄISCHE PATENTSCHRIFT

(45) Veröffentlichungstag der Patentschrift: **28.07.82**

(51) Int. Cl.³: **C 07 C 175/00,** C 07 D 317/28, A 61 K 31/16

(21) Anmeldenummer: **79103682.5**

(22) Anmeldetag: **28.09.79**

(54) **N-Monohydroxypropylamide, N-Dihydroxypropylamide und deren Acetonoide der all-E- und 13-Z-Retinsäure, Verfahren zu ihrer Herstellung und diese enthaltende pharmazeutische Mittel.**

(30) Priorität: **07.10.78 DE 2843870**

(43) Veröffentlichungstag der Anmeldung: **16.04.80 Patentblatt 80/8**

(45) Bekanntmachung des Hinweises auf die Patenterteilung: **28.07.82 Patentblatt 82/30**

(84) Benannte Vertragsstaaten: **AT BE CH DE FR GB IT LU NL SE**

(56) Entgegenhaltungen: **DE-A-2 102 586**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Paust, Joachim, Dr., Ringstrasse 3, D-6701 Neuhofen (DE)**
Erfinder: **Nuerrenbach, Axel, Dr., Koenigsberger Strasse 7, D-6718 Gruenstadt 1 (DE)**
Erfinder: **Koenig, Horst, Dr., Pariser Strasse 4, D-6700 Ludwigshafen (DE)**

**EP 0 009 776 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# N-Monohydroxypropylamide, N-Dihydroxypropylamide und deren Acetonoide der all-E- und 13-Z-Retinsäure, Verfahren zu ihrer Herstellung und diese enthaltende pharmazeutische Mittel

Die vorliegende Erfindung betrifft neue N-Monohydroxypropylamide, N-Dihydroxypropylamide und deren Acetonoide der all-E- und 13-Z-Retinsäure der allgemeinen Formeln I und II, in denen

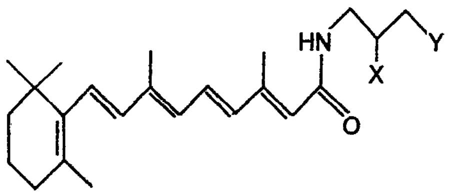

I

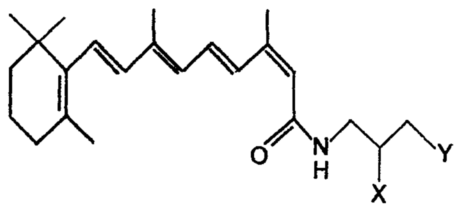

II

X und Y ein Wasserstoffatom oder eine Hydroxygruppe darstellen, wobei wenigstens einer der Reste X und Y eine Hydroxygruppe bedeuten, oder X und Y zusammen mit den sie verbindenden Kohlenstoffatomen einen 2,2-Dimethyl-1,3-dioxolanring bilden, die Herstellung dieser Verbindungen, diese enthaltende pharmazeutische Zubereitungen und ihre Verwendung als Arzneimittel.

Aus der DE-OS 2 102 586 ist bekannt, dass Retinsäureamide, die am Amidstickstoff beispielsweise durch Alkoxy- oder Alkylamino-substituierte Alkylreste oder eine Hydroxyniederalkylgruppe, eine Phenyl- oder Benzylgruppe als Substituenten aufweisen, pharmakologische Wirkungen zeigen.

Für diese Verbindungen wird beschrieben, dass sie zur topischen und systemischen Therapie von Karzinomen und Praekanzerosen sowie zur topischen und systemischen Prophylaxe von Karzinomen verwendet werden können. Sie lassen sich auch zur Therapie von Akne, Psoriasis und anderen mit verstärkter oder pathologisch veränderter Verhornung einhergehenden dermatologischen Affektionen sowie bei Ekzemen und bei Affektionen der Schleimhäute verwenden. In der DE-OS 2 102 586 werden u.a. beispielsweise als Einzelverbindungen Retinsäure-N-ethylamid und Retinsäure-N-(β-hydroxy)-ethylamid beschrieben.

Es wurde nun gefunden, dass die Verbindungen der Formel I und II besonders wertvolle pharmakologische Eigenschaften aufweisen.

Die erfindungsgemässen Verbindungen werden in an sich üblicher Weise hergestellt durch Umsetzung eines funktionellen Derivats der all-E- oder 13-Z-Retinsäure mit einem Amin der allgemeinen Formel III

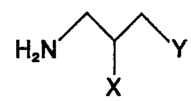

III,

in der X und Y die für Formeln I und II angegebenen Bedeutungen haben und die vorhandenen Hydroxygruppen zweckmässigerweise mit einer Schutzgruppe versehen sind, die anschliessend in üblicher Weise durch saure Hydrolyse abgespalten wird.

Als funktionelle Derivate der all-E- bzw. 13-Z-Retinsäure werden insbesondere die Säurehalogenide, vorzugsweise die Säurechloride, sowie die Ester von all-E- und 13-Z-Retinsäure, beispielsweise die Methyl- oder Phenylester verwendet.

Das 2- und 3-Hydroxy-propylamin-1 setzt man bevorzugt in Form der Tetrahydropyranyl-ether (IV, V), das 2,3-Dihydroxypropylamin-1 zweckmässigerweise in Form des Acetonids VI ein.

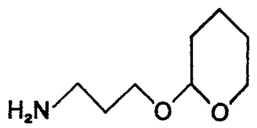

IV

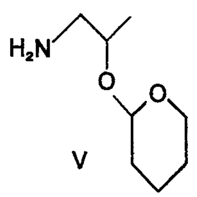

V

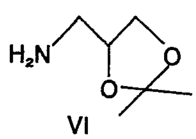

VI

Die Umsetzung wird vorzugsweise in einem inerten organischen Lösungsmittel, wie einem Dialkylether, einem gesättigten cyclischen Äther oder einem chlorierten aliphatischen Kohlenwasserstoff, beispielsweise Diethylether, Tetrahydrofuran oder Methylenchlorid, oder in Gemischen dieser Lösungsmittel bei Temperaturen von −20 bis 40 °C, bevorzugt von −20 bis 0 °C, durchgeführt. Zweckmässigerweise arbeitet man unter Ausschluss von Sauerstoff und Feuchtigkeit, z.B. unter Stickstoff als Inertgas.

Es ist zweckmässig, den bei der Reaktion eines Säurechlorids entstehenden Chlorwasserstoff mit einem HCl-Acceptor abzufangen. Dazu werden tertiäre Amine, wie Triäthylamin oder bevorzugt Pyridin, verwendet.

Die Abspaltung der Schutzgruppen erfolgt in an sich üblicher Weise, beispielsweise in schwefelsaurer Lösung, zweckmässig in einem wässrigen Tetrahydrofuran/Methanol-Gemisch bei einem pH-Wert von 1 bis 2 und bei Temperaturen von 30 bis 50 °C.

Die Aufarbeitung des Endprodukts erfolgt in an sich üblicher Weise; vorteilhaft beispielsweise säulenchromatographisch an Aluminiumoxid oder Kieselgel oder durch Umkristallisation.

Die erfindungsgemässen Verbindungen sind in ihrer biologischen Aktivität den bekannten Retinsäureamiden der DE-OS 2 102 586 in etwa vergleichbar. Beispielsweise heben sowohl Retinsäure-N-(β-hydroxy)-ethylamid als auch die erfindungsgemässen Retinoide die durch Vitamin A-Hypovitaminose induzierte Keratinisierung von Hamster-Tracheen-Gewebe noch in $10^{-8}$ molarer Lösung vollständig auf. Die Methodik hierzu kann G.H. Clamon et al Nature 250, 64–66 (1974) oder M.B. Sporn et al Nature 253, 47–50 (1975) entnommen werden. Die Keratinisierung wird als praekanzerotischer Prozess angesehen.

Die tumorhemmende Wirkung der erfindungsgemässen Verbindungen ist signifikant. Man beobachtet eine Wachstumskontrolle bei in vitro kultivierten Zellen, die beispielsweise nach der Methodik, wie sie von R. Lotan et al. im Journal of the National Cancer Institute 60 (1978) Seiten 1035–1041 beschrieben wird, nachgewiesen werden kann. Die erfindungsgemässen Verbindungen inhibieren die Proliferation von spontan chemisch oder durch Viren transformierter Zellen in Gewebekultur, vorzugsweise verwendet man die S 91 Melanoma Zellinie.

Die praktische Nützlichkeit von Retinoiden bei der Krebsprophylaxe und Therapie hängt jedoch keineswegs nur von der biologischen Aktivität ab. Entscheidend sind vielmehr ausserdem Toxizität und Pharmakokinetik der einzelnen Wirkstoffe in vivo. Beispielsweise kann es für die Anwendung eines Retinoids nachteilig sein, wenn dieses in bestimmten Organen im Verlauf der Behandlung in so hoher Konzentration gespeichert wird, dass toxische Nebenwirkungen auftreten. Anderseits kann die Konzentration eines Retinoids oder seiner aktiven Metaboliten in bestimmten von Praekanzerosen oder Karzinomen befallenen Organen so gering sein, dass die erwünschte Heilwirkung dort nicht eintritt. Die erfindungsgemässen Verbindungen I und II sind in diesen Punkten sowohl dem Retinsäure-N-(β-hydroxy)-ethylamid als auch den isomeren Retinsäure-N-hydroxybutylamiden, wie dem Retinsäure-N-(3,4-dihydroxy)-n-butylamid und dem -(3-hydroxy)- und -(4-hydroxy)-n-butylamid, eindeutig und in nicht vorhersehbarer Weise überlegen, d.h. dass sie eine wesentlich grössere therapeutische Breite aufweisen. Als bevorzugte Retinsäure-N-hydroxypropyl-amide sind die Dihydroxy-Verbindungen der Formel I (X = Y = OH) und II (X = Y = OH) sowie ihre Acetonide zu nennen.

Die erfindungsgemässen Verbindungen können aufgrund der genannten pharmakologischen Eigenschaften bei der topischen und systemischen Therapie und Prophylaxe von Praekanzerosen und Karzinomen der Haut, Schleimhäute und inneren Organe sowie bei der topischen und systemischen Therapie von Akne, Psoriasis und anderen mit pathologisch veränderter Verhornung einhergehenden dermatologischen Erkrankungen verwendet werden.

Ein bevorzugtes Indikationsgebiet ist die prophylaktische und therapeutische Behandlung von Praekanzerosen und Tumoren der Blase, der Brustdrüse, der Haut und der Schleimhäute.

Dementsprechend sind ein weiterer Gegenstand der Erfindung pharmazeutische Mittel, die eine Verbindung der Formel I oder II als Wirkstoff neben pharmazeutisch üblichen Trägerstoffen oder Verdünnungsmitteln und gegebenenfalls üblichen pharmazeutisch-technischen Hilfsstoffen enthalten, und die Verwendung einer Verbindung der Formel I oder II zur Herstellung eines Arzneimittels.

Zur vorliegenden Erfindung gehört auch die Herstellung der therapeutischen Mittel oder Zubereitungen, die mit üblichen Trägerstoffen oder Verdünnungsmitteln und gegebenenfalls üblichen pharmazeutisch-technischen Hilfsstoffen entsprechend der gewünschten Applikationsart mit einer zur Anwendung geeigneten Dosierung in an sich üblicher Weise, insbesondere durch Vermischen erfolgt.

Die therapeutischen Mittel enthalten die erfindungsgemäss zu verwendenden Verbindungen bei lokaler Anwendung in 0,001 bis 1,0%iger Konzentration, bevorzugt in 0,01 bis 0,1%iger Konzentration, und bei systemischer Anwendung vorzugsweise in einer Einzeldosis von 0,1 bis 5 mg. Dabei kommen als Tagesdosen 5 bis 100 mg in Betracht, wobei die Dosierungen je nach Art und Schwere der Erkrankung, der Zubereitung und der Applikation variieren können.

Es wurden die üblichen galenischen Zubereitungen verwendet, für die orale Applikation sind beispielsweise Tabletten, Filmtabletten, Dragees, Kapseln, Pillen, Pulver, Granulate, Lösungen oder Suspensionen geeignet. Für die äusserliche Anwendung kommen insbesondere Pasten, Salben, Gele, Cremes, Lotionen, Puder, Lösungen oder Emulsionen und Sprays in Betracht.

Die erfindungsgemässen Mittel gelangen insbesondere zur innerlichen und äusserlichen Anwendung. Sie werden vorzugsweise oral oder lokal appliziert.

Üblicherweise verwendete pharmazeutischtechnische Hilfsstoffe sind beispielsweise für die lokale Anwendung Alkohole, wie Isopropanol, oxäthyliertes Ricinusöl oder oxäthyliertes hydriertes Ricinusöl, Polyacrylsäure, Glycerinmonostearat, Parrafinöl, Vaseline, Wollfett, Polyäthylenglykol 400, Polyäthylenglykol 400-Stearat sowie äthoxylierter Fettalkohol, für die systemische Anwendung Saccharose, Milchzucker, Propylenglykol und Äthanol, Stärke, Talkum, Polyvinylpyrrolidon.

Weitere übliche Zusätze sind beispielsweise Konservierungsmittel, Antioxydantien, geschmackverbessernde Zusätze, Stabilisierungsmittel, Emulgiermittel, Gleitmittel, Netzmittel usw., wobei alle bei der Herstellung pharmazeutischer Zubereitungen verwendeten Stoffe untoxisch und mit den verwendeten Wirkstoffen verträglich sein sollen (vgl. L.G. Goodman, A. Gilman, The Pharmacological Basis of Therapeutics).

Die (Isomeren)-Reinheit der hergestellten erfindungsgemässen Verbindungen lässt sich HPLCanalytisch bestimmen und liegt in der Regel >96%. Die Strukturanordnung basiert auf der H-NMR-Spektroskopie.

Beispiel 1

N-Formyl-3-hydroxy-propylamin-1:

60,5 Gewichtsteile Ameisensäure-methylester werden innerhalb von 5 min unter Rühren und

Kühlung mit einem Wasserbad zu 75,1 Gewichtsteilen 3-Hydroxy-propylamin-1 getropft. Die Temperatur steigt dabei auf ca. 70 °C an. Anschliessend wird 1 h bei 80 °C gerührt. Bei der anschliessenden Destillation erhält man 75,5 Gewichtsteile N-Formyl-3-hydroxypropyl-amin-1, Kp 134–137 °C (0,027 mbar).

2-(3-N-Formylamino-propoxy)-tetrahydro-2H-pyran:

Zu einer Lösung von 103,0 Gewichtsteilen N-Formyl-3-hydroxy-propylamin-1 in 220 Raumteilen Diethylether und 220 Raumteilen Methylenchlorid gibt man 171,7 Gewichtsteile 3,4-Dihydro-2H-pyran und leitet in diese Lösung bei Raumtemperatur 2 min lang gasförmigen Chlorwasserstoff ein. Die Mischung wird 1 h auf Rückflusstemperaturen erhitzt, auf Raumtemperatur abgekühlt und mit 450 Raumteilen 5gewichtsprozentiger wässriger Natrium-bicarbonatlösung versetzt. Nach üblicher Aufarbeitung der organischen Phase erhält man 135 Gewichtsteile 2-(3-Formylamino-propoxy)-tetrahydro-2H-pyran, Kp 122 °C (0,013 mbar).

2-(3-Amino-propoxy)-tetrahydro-2-H-pyran:

187,2 Gewichtsteile 2-(3-N-Formylamino-propoxy)-tetrahydro-2H-pyran werden mit 132 Gewichtsteilen 85prozentigem Kaliumhydroxid und 850 Raumteilen Ethanol versetzt und 30 min lang auf Rückflusstemperatur erhitzt. Man kühlt ab, gibt 650 Raumteile Wasser zu und extrahiert viermal mit je 500 Raumteilen Diethylether. Die Etherlösung wird mit Wasser gewaschen und über Kaliumcarbonat getrocknet und wie üblich aufgearbeitet. Durch fraktionierende Destillation erhält man 119 Gewichtsteile 2-(3-Aminopropoxy)-tetrahydro-2H-pyran, Kp 104–106 °C (16 mbar), Gehalt nach gaschromatographischer Analyse 92%.

Retinsäure-N-3-(2-tetrahydro-2H-pyranyloxy-)-n-propylamid:

Man suspendiert 120 Gewichtsteile all-E-Retinsäure in 1600 Raumteilen Diethylether und versetzt mit 34 Raumteilen Pyridin. Bei −5 °C wird eine Lösung von 32,5 Raumteilen Thionylchlorid in 200 Raumteilen Diethylether zugetropft und noch 2 h im Eisbad nachgerührt. Man saugt unter Ausschluss von Sauerstoff und Feuchtigkeit ab, wäscht das Kristallisat (Pyridinium-hydrochlorid) mit 200 Raumteilen Diethylether und lässt das eisgekühlte Filtrat so in eine Lösung von 69,2 Gewichtsteilen 2-(3-Amino-propoxy)-tetrahydro-2H-pyran und 34 Raumteilen Pyridin in 300 Raumteilen Diethylether einfliessen, dass die Temperatur −20 °C nicht überschreitet. Die Mischung wird 2 h ohne Kühlung nachgerührt. Man lässt dann absetzen, dekantiert die überstehende Lösung ab, kocht den Rückstand 10 min lang in 500 Raumteilen Diisopropylether auf, lässt wiederum absetzen und dekantiert erneut. Ether- und Diisopropyletherphase werden vereinigt, zweimal mit kalter 2,5prozentiger Natriumbicarbonatlösung gewaschen und wie üblich aufgearbeitet. Man erhält 117,9 Gewichtsteile rohes Retinsäure-N-3-(2-tetrahydro-2H-pyranyloxy)-n-propylamid, das bei −10 °C aus 350 Raumteilen Diisopropylether umkristallisiert wird: 92,2 Gewichtsteile, Fp. 73–74 °C.

all-E-Retinsäure-N-(3-hydroxy)-propylamid:

82,2 Gewichtsteile Retinsäure-N-3-(2-tetrahydro-2H-pyranyloxy)-n-propylamid werden in 1000 Raumteilen 80prozentigem wässrigem Tetrahydrofuran und 100 Raumteilen Methanol gelöst, mit 10 Raumteilen 65prozentiger Schwefelsäure auf pH 2 gestellt und 30 min bei 45 °C gerührt. Zur Aufarbeitung gibt man 800 Gewichtsteile Eis zu und extrahiert viermal mit je 200 Raumteilen Methylenchlorid, wäscht die organische Phase mit 500 Raumteilen kalter 2,5prozentiger Natrium-bicarbonatlösung, arbeitet wie üblich auf und erhält 102 Gewichtsteile Rohprodukt, die bei 0 °C aus 3 l Diisopropylether umkristallisiert werden: 55 Gewichtsteile Retinsäure-N-(3-hydroxy)-propylamid, Fp. 109–110 °C.

Beispiel 2

13-Z-Retinsäure-N-3-(2-tetrahydro-2H-pyranyl-oxy)-propylamid:

Wie in Beispiel 1 angegeben, werden 60 Gewichtsteile 13-Z-Retinsäure in das Säurechlorid übergeführt und mit 34,6 Gewichtsteilen 2-(3-Aminopropoxy)-tetrahydro-2H-pyran umgesetzt. Man erhält 60,6 Gewichtsteile Rohprodukt, das an 800 Gewichtsteilen basischem Aluminiumoxid chromatographiert wird (Akt. Stufe III, Diisopropylether). Das Einengen der nach GC-Analyse reinen Fraktionen liefert 48 Gewichtsteile 13-Z-Retinsäure-N-3-(2-tetrahydro-2H-pyranyloxy)-propylamid als gelbes Öl, dessen Struktur bzw. Konfiguration H-NMR-spektroskopisch sichergestellt wurde. Charakteristisch sind beispielsweise die chemischen Verschiebungen der $C_{20}$-Methylgruppe (2,0 ppm) und des $C_{14}$-Vinylprotons (5,45 ppm) im Vergleich zu den entsprechenden Signalen der all-E-Verbindung (2,3 ppm bzw. 5,65 ppm).

13-Z-Retinsäure-N-(3-hydroxy)-propylamid:

Nach der in Beispiel 1 für die all-E-Verbindung angegebenen Vorschrift werden 40 Gewichtsteile 13-Z-Retinsäure-N-3-(2-tetrahydro-2H-pyranyloxy)-propylamid der säurekatalysierten Hydrolyse unterworfen. Das Rohprodukt wird an 900 Gewichtsteilen Kieselgel chromatographiert (Diisopropylether-Essigester): 29 Gewichtsteile 13-Z-Retinsäure-N-(3-hydroxy)-propylamid, gelbes Öl. Struktur bzw. Konfiguration werden durch die H-NMR-Analyse bestätigt: $C_{20}$-Methylprotonen 2,0 ppm, $C_{14}$-Vinylproton 5,45 ppm.

Beispiel 3

α-(2-Tetrahydro-2H-pyranyloxy)-propionitril:

71,1 Gewichtsteile Acetaldehyd-cyanhydrin werden in 200 Raumteilen Methylenchlorid gelöst, mit 0,2 Raumteilen konzentrierter wässriger Salzsäure und 168 Gewichtsteilen Dihydropyran versetzt (Temperaturanstieg bis 50 °C) und anschliessend 3 h bei 25 °C gerührt.

Zu dieser Lösung gibt man 1000 Raumteile 5gewichtsprozentige Sodalösung und extrahiert

dreimal mit je 300 Raumteilen Methylenchlorid. Man arbeitet wie üblich auf und rektifiziert den Rückstand bei Kp 48–49 °C (0,4 mbar), 138 Gewichtsteile α-(2-Tetrahydro-2H-pyranyloxy)-propionitril; nach GC-Analyse ein Gemisch von zwei Diastereomeren im Verhältnis 1,25:1.

2-(2-Tetrahydro-2H-pyranyloxy)-propyl-1-amin:

Zu einer Suspension von 27 Gewichtsteilen Lithium-aluminium-hydrid in 1200 Raumteilen Diethylether tropft man innerhalb von 35 min unter schwachem Sieden eine Lösung von 129,5 Gewichtsteilen α-(2-Tetrahydro-2H-pyranyloxy)-propionitril in 250 Raumteilen Diethylether, kocht danach 2 h unter Rückfluss und hydrolysiert das überschüssige Reduktionsmittel durch Zugabe von Eis. Die Etherphase wird durch Absaugen von Aluminiumhydroxid abgetrennt und wie üblich aufgearbeitet, wobei 116,4 Gewichtsteile Rohprodukt erhalten werden. Durch Rektifizieren gewinnt man bei Kp 60–64 °C (0,53 mbar) 104,5 g 2-(2-Tetrahydro-2H-pyranyloxy)-propyl-1-amin mit einem Reinheitsgrad von 96%.

all-E-Retinsäure-N-(2-tetrahydro-2H-pyranyloxy)-propylamid:

Eine nach Beispiel 1 aus 120 Gewichtsteilen Retinsäure hergestellte etherische Lösung von Retinsäurechlorid gibt man so zu einer Lösung von 66,9 Gewichtsteilen 2-(2-Tetrahydro-2H-pyranyloxy)-propyl-1-amin und 34 Raumteilen Pyridin in 500 Raumteilen Diethylether, dass die Temperatur −20 °C nicht übersteigt und lässt den Ansatz dann innerhalb von 2 h auf 25 °C sich erwärmen. Man setzt 600 Raumteile n-Hexan zu und wäscht zweimal mit je 300 Raumteilen 5gewichtsprozentiger Lösung von Natriumhydrogencarbonat. Aus der organischen Phase isoliert man in üblicher Weise 109 Gewichtsteile Rohprodukt, die in 1000 Raumteilen Diisopropylether gelöst und zur Abtrennung von Retinsäure an 400 Gewichtsteilen Aluminiumoxid (Akt. – Stufe III) filtriert werden. Das Filtrat wird eingeengt und liefert 88 Gewichtsteile all-E-Retinsäure-N-2-(2-tetrahydro-2H-pyranyloxy)-propylamid als gelbes Öl, das nach gaschromatographischer und H-NMR-spektroskopischer Analyse rein ist.

all-E-Retinsäure-N-(2-hydroxy)-propylamid:

80 Gewichtsteile Retinsäure-N-2-(2-tetrahydro-2H-pyranyloxy)-propylamid werden in 900 Raumteilen 80%igem wässrigem Tetrahydrofuran und 100 Raumteilen Methanol gelöst, mit 20 Raumteilen 65prozentiger Schwefelsäure auf pH 2 gebracht und 1 h bei 45 °C gerührt. Man kühlt auf 20 °C ab, gibt 800 Gewichtsteile Eis zu, extrahiert dreimal mit je 350 Raumteilen Methylenchlorid, wäscht die organische Phase mit 100 Raumteilen 2,5gewichtsprozentiger Natriumhydrogencarbonatlösung und arbeitet in üblicher Weise auf. Dabei fallen 81 Gewichtsteile Rohprodukt an, die nach der Kristallisation aus 150 Raumteilen Diisopropylether bei 0 °C 51 Gewichtsteile all-E-Retinsäure-N-(2-hydroxy)-propylamid liefern; Fp 115,8 °C. Aus der Mutterlauge lassen sich nach dem Einengen und Chromatographie an 800 Gewichtsteilen Aluminiumoxid (Akt.- Stufe III, Diisopropylether-Essigester) weitere 15 Gewichtsteile Wirkstoff isolieren.

Beispiel 4

13-Z-Retinsäure-N-2-(2-tetrahydro-2H-pyranyloxy)-propylamid:

Wie in Beispiel 3 angegeben, werden 60 Gewichtsteile 13-Z-Retinsäure in das Säurechlorid übergeführt und mit 33,5 Gewichtsteilen 2-(2-Tetrahydro-2H-pyranyloxy)-propylamin umgesetzt. Man erhält 54,5 Gewichtsteile Rohprodukt, die durch Chromatographieren an 500 Gewichtsteilen basischem Aluminiumoxid (Akt.-Stufe III, Diisopropylether) gereinigt werden: 46 Gewichtsteile 13-Z-Retinsäure-N-2-(2-tetrahydro-2H-pyranyloxy)-propylamid, gelbes Öl. Die Struktur wird durch H-NMR-Spektroskopie gestützt: $C_{20}$-Methylprotonen 2,05 ppm, $C_{14}$-Vinylprotonen 5,5 ppm.

13-Z-Retinsäure-N-(2-hydroxy)-propylamid:

Wie in Beispiel 3 beschrieben, werden 40 Gewichtsteile 13-Z-Retinsäure-N-2-(2-tetrahydro-2H-pyranyloxy)propylamid der säurekatalysierten Hydrolyse unterworfen. Das Rohprodukt (38 Gewichtsteile) wird durch Säulenchromatographie an 800 Gewichtsteilen Kieselgel (Diisopropylether-Essigester) gereinigt: 29 Gewichtsteile 13-Z-Retinsäure-N-(2-hydroxy)-propylamid, gelbes Öl. Struktur bzw. Konfiguration werden durch H-NMR-Spektroskopie bewiesen: $C_{20}$-Methylprotonen 2,05 ppm, $C_{14}$-Vinylprotonen 5,5 ppm.

Beispiel 5

1-Formylamino-2,3-dihydroxy-propan:

Zu 91,1 Gewichtsteilen 1-Amino-2,3-dihydroxy-propan gibt man tropfenweise 121 Gewichtsteile Ameisensäuremethylester. Wenn die exotherme Umsetzung nachlässt, erhitzt man noch 30 min unter Rückfluss und destilliert den überschüssigen Ameisensäuremethylester im Rotationsverdampfer ab.

N-(2,2-Dimethyl-1,3-dioxolan-4-yl)-methyl-formamid:

119 Gewichtsteile 1-Formylamino-2,3-dihydroxy-propan werden in 214 Raumteilen Dimethylformamid gelöst und mit 1,8 Gewichtsteilen p-Toluolsulfonsäure und 210 Gewichtsteilen 2,2-Dimethoxypropan versetzt. Man rührt 30 min bei 50 °C und lässt dann über Nacht stehen. Die Lösung wird bei ca. 4 mbar am Rotationsverdampfer eingeengt und der Rückstand nach dem Entgasen bei Kp 80 °C und 2 mbar einer Kurzwegdestillation unterworfen (Kp 145 °C) 0,027 mbar. Dabei erhält man 109 Gewichtsteile N-2,2-Dimethyl-1,3-dioxolan-4-yl)-methyl-formamid, das nach GC-Analyse einen Gehalt von 96% aufweist.

2,2-Dimethyl-4-aminomethyl-1,3-dioxolan:

Man löst 131,7 Gewichtsteile 85prozentiges Kaliumhydroxid in 900 Raumteilen Methanol, gibt 151,5 Gewichtsteile N-(2,2-Dimethyl-1,3-dioxolan-4-yl)-methyl-formamid zu und kocht die Mischung 45 min unter Rückfluss. Anschliessend wird das Methanol über eine Vigreux-Kolonne bis zu einer Übergangstemperatur von 80 °C abdestilliert. Den Rückstand trennt man zwischen Diethylether und gesättigter Kochsalzlösung, engt ein und unterwirft den Rückstand einer fraktionierenden Destillation. Bei Kp 72–73 °C (33,25 mbar) gehen 105 Gewichtsteile 2,2-Dimethyl-4-amino-methyl-1,3-dioxolan über.

Retinsäure-N-(2,2-dimethyl-1,3-dioxolan-4-yl)-methylamid:

Wie in Beispiel 1 beschrieben, stellt man aus 180 Gewichtsteilen all-E-Retinsäure eine etherische Lösung von Retinsäurechlorid her. Diese Lösung wird so zu einer Lösung von 67,5 Gewichtsteilen 2,2-Dimethyl-4-aminomethyl-1,3-dioxolan und 51 Raumteilen Pyridin in 624 Raumteilen Diethylether gegeben, dass die Temperatur −20 °C nicht übersteigt. Man lässt das Gemisch innerhalb 1 h auf 25 °C sich erwärmen, setzt 1000 Raumteile eiskalte 2,5gewichtsprozentige Natriumhydrogencarbonatlösung zu und arbeitet in üblicher Weise auf. Das Rohprodukt (245 Gewichtsteile) wird zur Abtrennung von unpolaren Verunreinigungen und Retinsäure an 1000 Gewichtsteilen basischem Aluminiumoxid chromatographiert (Akt.-Stufe III, Diisopropylether-Essigester). Die nach GC-Analyse sauberen Fraktionen ergeben zusammen 187 Gewichtsteile all-E-Retinsäure-N-(2,2-dimethyl-1,3-dioxolan-4-yl)-methylamid. Eine Probe zeigt nach Kristallisation an Diisopropylether einen Schmelzpunkt von 106 °C.

all-E-Retinsäure-N-(2,3-dihydroxy-)propylamid:

50 Gewichtsteile Retinsäure-N-(2,2-dimethyl-1,3-dioxolan-4-yl)-methylamid werden in 700 Raumteilen 80prozentigem wässrigem Tetrahydrofuran und 100 Raumteilen Methanol gelöst, mit 4 Raumteilen 65prozentiger Schwefelsäure auf pH 1,5 gestellt und 30 min bei 45 °C gerührt. Man gibt 500 Gewichtsteile Eis zu und arbeitet in üblicher Weise durch Trennen zwischen Ether und Wasser auf. Das Rohprodukt (52 Gewichtsteile) wird zur Abtrennung geringer Mengen unpolarer Verunreinigungen und Retinsäure an 400 Gewichtsteilen Kieselgel chromatographiert (Diisopropylether-Essigester). Das Produkt aus den nach GC-Analyse reinen Fraktionen (44 Gewichtsteile) wird bei −15 °C aus Essigester kristallisiert: 31,5 Gewichtsteile all-E-Retinsäure-N-(2,3-dihydroxy)-propylamid, Fp. 96 °C.

Beispiel 6

13-Z-Retinsäure-N-(2,2-Dimethyl-1,3-dioxolan-4-yl)-methylamid:

Wie in Beispiel 5 beschrieben, stellt man aus 60 Gewichtsteilen 13-Z-Retinsäure und 22,5 Gewichtsteilen 2,2-Di-methyl-4-aminomethyl-1,3-dioxolan zunächst 81 Gewichtsteile rohes 13-Z-Retinsäure-N-(2,2-dimethyl-1,3-dioxolan-4-yl)-methylamid her. Zur Reinigung wird das Produkt an 800 Gewichtsteilen basischem Aluminiumoxid chromatographiert (Akt.-Stufe III, Diisopropylether-Essigester): Ausbeute 66 Gewichtsteile, gelbes Öl. H-NMR-Daten: $C_{20}$-Methylprotonen 2,0 ppm, $C_{14}$-Vinylprotonen 5,5 ppm.

13-Z-Retinsäure-N-(2,3-dihydroxy)-propylamid:

Wie in Beispiel 5 beschrieben, werden 25 Gewichtsteile 13-Z-Retinsäure-N-(2,2-dimethyl-1,3-dioxolan-4-yl)-methylamid der säurekatalysierten Hydrolyse unterworfen; Rohausbeute 22 Gewichtsteile. Das Produkt wird durch Säulenchromatographie an 600 Gewichtsteilen Kieselgel gereinigt (Essigester-Methanol). Man erhält 18 Gewichtsteile 13-Z-Retinsäure-N-(2,3-dihydroxy)-propylamid als gelbes Öl. Charakteristische H-NMR-Daten: $C_{20}$-Methylprotonen bei 2,0 ppm, $C_{14}$-Vinylprotonen bei 5,45 ppm.

Geeignete pharmazeutische Zubereitungen oder Arzneistoffträger für die äusserliche Anwendung sind z.B.:

Beispiel 1

Lösung

| | |
|---|---|
| all-E-Retinsäure-N-(2,3-dihydroxy)-propylamid | 0,25 g |
| oxäthyliertes hydriertes Ricinusöl (Cremophor RH 40, Hersteller BASF AG, Ludwigshafen) | 35,0 g |
| Polyäthylenglykol 400 | 35,0 g |
| oxäthyliertes Ricinusöl (Softigen 767, Hersteller Chemische Werke, Witten) | 10,0 g |
| demineralisiertes Wasser | ad 100,0 g |

Cremophor RH 40 und Softigen 767 werden gemischt und auf 70 °C erhitzt. Die Wirksubstanz wird unter Rühren gelöst und Polyäthylenglykol 400 zugesetzt. Die Lösung wird dann auf 40 °C abgekühlt und unter Rühren wird langsam auf 40 °C erhitztes Wasser zugesetzt. Die fertige Lösung wird filtriert und in z.B. 100 ml Flaschen abgefüllt.

Beispiel 2

Creme

| | |
|---|---|
| 13-Z-Retinsäure-N-(2,3-dihydroxy)-propylamid | 0,1 g |
| Butylhydroxytoluol | 0,1 g |
| Glycerinmonostearat | 11,0 g |
| Polyäthylenglykol 400-Stearat | 6,0 g |
| äthoxylierter Fettalkohol | 4,0 g |
| Parrafinöl | 10,0 g |
| p-Hydroxybenzoesäureester (Nipasteril, Hersteller Nipalaboratorium Hamburg) | 0,2 g |
| Parfümöl | 0,1 g |
| demineralisiertes Wasser | ad 100,0 g |

Die Fette werden geschmolzen und die feinstgepulverte Wirksubstanz sowie Butylhydroxytoluol unter Rühren bei 65 °C darin verteilt (Lösung I). Das Wasser wird mit dem Nipaester aufgekocht und auf 65 °C abgekühlt (Lösung II). In kleinen Anteilen wird Lösung II unter gutem Rühren in Lösung I einemulgiert. Nach dem Abkühlen auf 45 °C wird das Parfümöl zugesetzt und die Emulsion unter Rühren auf Zimmertemperatur abgekühlt. Die fertige Creme wird in innenschutzlakkierte Tuben abgefüllt.

Beispiel 3
Gel

| | |
|---|---|
| all-E-Retinsäure-N-(3-hydroxy)-propylamid | 0,01 g |
| Butylhydroxytoluol | 0,1 g |
| oxäthyliertes Ricinusöl (Cremophor EL, Hersteller BASF AG, Ludwigshafen) | 35,0 g |
| Isopropanol | 20,0 g |
| Polyacrylsäure (Carbopol, Hersteller Goodrich Hamburg) | 1,5 g |
| Triäthanolamin | 0,002 g |
| p-Hydroxybenzoesäureester (Nipasteril, Hersteller Nipalaboratorium Hamburg) | 0,2 g |
| demineralisiertes Wasser | ad 100,0 g |

Das Cremophor EL wird auf 60 °C erhitzt, der Wirkstoff und das Butylhydroxytoluol unter Rühren gelöst und das Isopropanol, in dem die Nipaester gelöst wurden, zugemischt (Lösung I). Carbopol wird unter kräftigem Rühren in dem Wasser verteilt (Lösung II). Lösung II wird unter gutem Rühren in kleinen Anteilen zu Lösung I gemischt. Der pH-Wert des Gemisches wird mit Triäthanolamin auf 4,5 eingestellt. Das fertige Gel wird in innenschutzlackierte Tuben abgefüllt.

Für die systemische Anwendung besonders geeignete Zubereitungen oder Arzneistoffträger sind z.B.:

Beispiel 4
Tropfen

| | |
|---|---|
| all-E-Retinsäure-N-(2,3-dihydroxy)-propylamid | 0,1 g |
| Propylenglykol | 25,0 g |
| Ethylalkohol | ad 50,0 g |

Ethylalkohol und Propylenglykol werden miteinander gemischt und die Wirksubstanz unter Erwärmen auf 35 °C und unter Rühren gelöst. Nach Filtration wird die Lösung in dunkle Tropfflaschen abgefüllt.

Beispiel 5
Hartgelatinekapseln

| | |
|---|---|
| 13-Z-Retinsäure-N-(2,3-dihydroxy)-propylamid | 1 mg |
| Milchzucker | ad 0,25 g |

Die Bestandteile werden gesiebt, gemischt und auf einer geeigneten Kapselfüll- und -verschlussmaschine in Hartgelatinekapseln der Grösse 2 gefüllt.

**Patentansprüche**

1. N-Monohydroxypropylamide, N-Dihydroxypropylamide und deren Acetonoide der all-E- und 13-Z-Retinsäure der Formeln I und II

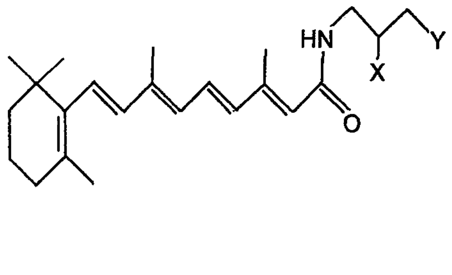

I

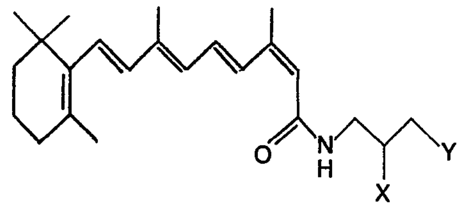

II

in denen X und Y ein Wasserstoffatom oder eine Hydroxygruppe darstellen, wobei wenigstens einer der Reste X oder Y eine Hydroxygruppe bedeutet, oder X und Y zusammen mit den sie verbindenden Kohlenstoffatomen einen 2,2-Dimethyl-1,3-dioxolanring bilden.

2. all-E-Retinsäure-N-(2,3-dihydroxy)-propylamid.

3. 13-Z-Retinsäure-N-(2,3-dihydroxy)-propylamid.

4. Verfahren zur Herstellung von Verbindungen der Formeln I und II nach Anspruch 1, dadurch gekennzeichnet, dass man ein funktionelles Derivat der all-E- oder 13-Z-Retinsäure mit einem Amin der Formel III

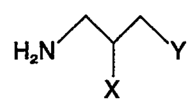

III,

in der X und Y die in Anspruch 1 angegebenen Bedeutungen haben und die vorhandenen Hydroxygruppen zweckmässig mit einer Schutzgruppe versehen sind, in an sich üblicher Weise umsetzt und gegebenenfalls anschliessend die Schutzgruppe durch saure Hydrolyse abspaltet.

5. Pharmazeutisches Mittel, gekennzeichnet durch einen Gehalt an einer Verbindung der Formeln I oder II nach Anspruch 1 als Wirkstoff neben üblichen Trägerstoffen oder Verdünnungsmitteln und gegebenenfalls pharmazeutisch-technischen Hilfsstoffen.

6. Pharmazeutisches Mittel nach Anspruch 5, gekennzeichnet durch einen Gehalt an all-E-Retinsäure-N-(2,3-dihydroxy)-propylamid.

7. Pharmazeutisches Mittel nach Anspruch 5, gekennzeichnet durch einen Gehalt an 13-Z-Retinsäure-N-(2,3-dihydroxy)-propylamid.

8. Verbindungen der Formeln I oder II nach Anspruch 1 zur Anwendung bei der topischen und systemischen Therapie und Prophylaxe von Praekanzerosen und Karzinomen der Haut, Schleimhäute und inneren Organe und bei dermatologischen Erkrankungen.

**Claims**

1. N-monohydroxypropylamides and N-dihydroxypropylamides of all-E-retinic acid and 13-Z-retinic acid, of the formulae I and II

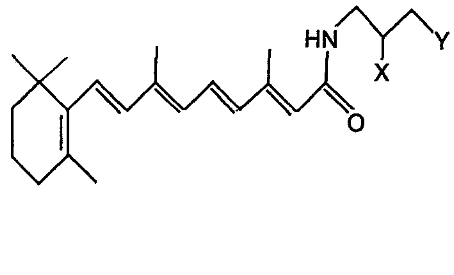

I

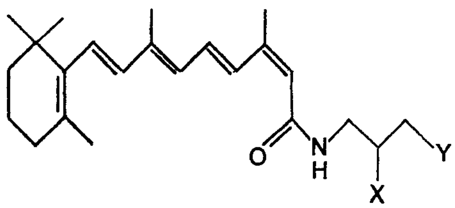

II

where X and Y are hydrogen or hydroxyl, but at least one is hydroxyl, or X and Y together with the carbon atoms linking them form a 2,2-dimethyl-1,3-dioxolane ring, and the acetonoids of such N-dihydroxypropylamides.

2. all-E-Retinic acid N-(2,3-dihydroxy)-propylamide.

3. 13-Z-Retinic acid N-(2,3-dihydroxy)-propylamide.

4. A process for the preparation of a coupound of the formula I or II as claimed in claim 1, characterized in that a functional derivative of all-E-retinic acid or of 13-Z-retinic acid is reacted in the conventional manner with an amine of the formula III

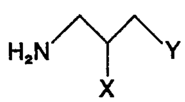

III,

where X and Y have the meanings given in claim 1 and the hydroxyl groups present are advantageously provided with a protective group, after which, where required, the protective group is removed by acid hydrolysis.

5. A pharmaceutical formulation characterized by a content of a compound of the formula I or II as claimed in claim 1 as the active ingredient, together with conventional carriers or diluents and with or without pharmaceutical auxiliaries.

6. A pharmaceutical formulation as claimed in claim 5, characterized by a content of all-E-retinic acid N-(2,3-dihydroxy)-propylamide.

7. A pharmaceutical formulation as claimed in claim 5, characterized by a content of 13-Z-retinic acid N-(2,3-dihydroxy)-propylamide.

8. A compound of the formula I or II as claimed in claim 1 for use in the tonical and systemic therapy and prophylaxis of pre-cancerous conditions and carcinomas of the skin, mucous membranes and internal organs, and of dermatological disorders.

**Revendications**

1. N-monohydroxypropylamide, N-dihydroxypropylamide et leurs acétonoides des acides all-E- et 13-Z-rétinoique des formules I et II

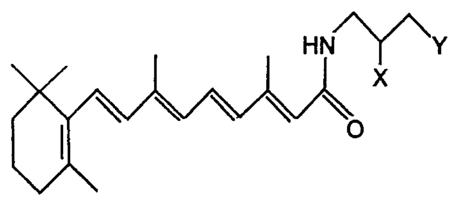

I

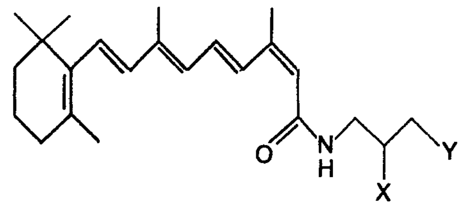

II

dans lesquelles X et Y représentent un atome d'hydrogène ou un groupe hydroxy, au moins un des restes X ou Y représentant un groupe hydroxy, ou X et Y forment, avec les atomes de carbone qui les relient, un noyau 2,2-diméthyl-1,3-dioxolane.

2. N-(2,3-dihydroxy)-propylamide d'acide all-E-rétinoique.

3. N-(2,3-dihydroxy)-propylamide d'acide 13-Z-rétinoique.

4. Procédé de préparation de composés de formules I et II selon la revendication 1, caractérisé par le fait que l'on fait réagir, de manière usuelle en soi, un dérivé fonctionnel des acides all-E ou 13-Z-rétinoiques avec une amine de formule III

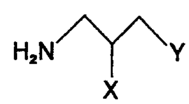

III,

dans laquelle X et Y ont les significations données à la revendication 1 et les groupes hydroxy présents sont avantageusement dotés d'un groupe protecteur, et on sépare éventuellement ensuite le groupe protecteur par hydrolyse acide.

5. Agent pharmaceutique, caractérisé par le fait qu'il contient un composé de formules I ou II selon la revendication 1 comme principe actif à côté de véhicules ou diluants usuels et éventuellement d'auxiliaires de la technique pharmaceutique.

6. Agent pharmaceutique selon la revendication 5, caractérisé par le fait qu'il contient un N-(2,3-dihydroxy)-propylamide d'acide all-E-rétinoique.

7. Agent pharmaceutique selon la revendication 5, caractérisé par le fait qu'il contient un N-(2,3-dihydroxy)-propylamide d'acide 13-Z-rétinoique.

8. Composés des formules I ou II selon la revendication 1 pour utilisation dans la thérapeutique et la prophylaxie locales et systémiques de précanceroses et carsinomes de la peau, muqueuse et organes internes et pour les maladies dermatologiques.